# EUROPEAN PATENT SPECIFICATION

(11) **EP 1 076 554 B1**
(45) Date of publication and mention of the grant of the patent: **13.10.2004**
(21) Application number: 99924193.8
(22) Date of filing: 12.05.1999
(51) Int. Cl.: A61K 7/50

(54) **CLEAR PERSONAL CARE FORMULATIONS CONTAINING QUATERNARY AMMONIUM COMPOUNDS AND OTHER NITROGEN-CONTAINING COMPOUNDS**
KLARE KÖRPERPFLEGE ZUBEREITUNGEN ENTHALTEND QUATERNÄRE AMMONIUM VERBINDUNGEN UND ANDERE STICKSTOFF-ENTHALTENDE VERBINDUNGEN
FORMULATIONS CLAIRES DE SOINS PERSONNELS CONTENANT DES COMPOSES D'AMMONIUM QUATERNAIRES ET D'AUTRES COMPOSES CONTENANT DE L'AZOTE

(30) Priority: 12.05.1998 US 76655
(43) Date of publication of application: 21.02.2001
(73) Proprietor: Goldschmidt Chemical Company, Hopewell, VA 23860 (US); Witco Surfactants GmbH, 36396 Steinau an der Strasse (DE)
(72) Inventor: DALRYMPLE, Damon, M., Columbus, OH 43202 (US); MANNING, Monna, Columbus, OH 43205 (US); MERZ, Frank, D-36396 Steinau (DE)
(74) Representative: Grünecker, Kinkeldey, Stockmair & Schwanhäusser Anwaltssozietät
(86) International application number: PCT/US1999/010405
(87) International publication number: WO 1999/058106

(56) References cited:
- EP-A- 0 559 894
- WO-A-97/03648
- US-A- 4 069 347

## Description

### BACKGROUND OF THE INVENTION

The present invention relates to formulations containing selected quaternary ammonium compounds that are useful for commercial products, for instance, personal care products such as hair conditioners, shampoos, skin conditioners. More particularly, the invention relates to the use of selected quaternary ammonium compounds to make clear and thick formulations attractive and useful for commercial products.

Although a variety of quaternary ammonium compounds or "quats" have been used and proposed for use in personal care products, many have drawbacks. For example, light color and low odor are essential to obtaining customer acceptance and to achieving stable and acceptable long-term product aesthetic properties. Indeed, there is increasing interest in obtaining personal care product formulations which are translucent or transparent (that is, clear) liquids, even to the point of obtaining a crystal clear dispersion. Discovery of such clear compositions requires careful identification of proper quaternary and/or polyquaternary ammonium compounds, together with appropriate additives, such as surfactants and solvents, which act together to achieve the desired appearance. Such clear personal care products would include hair conditioners, shampoos, skin conditioner, body washes, liquid bath soaps, facial cleansers, make-up removers, baby baths, and hand soaps.

Each of these applications presents its own complications, because the interactions between the various components of the compositions must be considered in addition to the individual chemistry of each component. For example, consider the formulation of a combined conditioner and shampoo composition, also known as conditioning shampoos, two-in-one shampoos, or two-in-ones. Two-in-ones contain ingredients that both clean and condition the hair in one application and are thus very time efficient. Two-in-ones, however, are traditionally pearlescent or opaque because most conditioning ingredients, for example, quaternary ammonium compounds, are not soluble in the anionic detergent carrier in the normal formulation parameters for these products. The detergent compounds with the widest range of cleaning properties are generally anionic (negatively charged) surfactants. Such anionic surfactants, for example, may include the alkylbenzene sulfonates, α-olefin sulfonates, sodium lauryl ether sulfates, sodium lauryl sulfates, ammonium lauryl ether sulfates, and ammonium lauryl sulfates available from Witco Corporation under the WITCOLATE® and WITCONATE® trademarks. In contrast, as exemplified by the quaternary ammonium compounds discussed above, conditioners are generally cationic (positively charged). Thus, when the anionic detergent ingredients and cationic conditioning ingredients are present in the same aqueous solution, as in two-in-ones, they have a natural tendency to complex together or even precipitate out of solution. This complexation or dispersion usually results in a pearlescent or opaque mixture and interferes with the performance of both the detergent compounds and conditioning compounds and is therefore undesirable.

UK Patent No. 2 160 421 relates to a conditioning and softening composition that include certain quaternary ammonium compounds. All of the examples prepared include coconut diethanolamide, a well-known thickening agent or viscosity-enhancing agent. The instant invention does not require diethanolamide or other conventional thickening agents or viscosity control agents, since the instant invention provides a clear formulation with a high viscosity from the components specified.

EP 0 511 652 A1 relates to a hair shampoo-conditioner formulation including an anionic cleansing surfactant, a polymeric cationic conditioning compound. a cationic conditioning surfactant, a fatty ester, and water. In contrast to the formulations of the instant invention which are clear and have high viscosities, the compositions of EP 0 511 652 A1 are all opaque to pearlescent formulations (Examples 1 and 3-10), often with low viscosities. In addition, the compositions of EP 0 511 652 A1 require a polymeric cationic conditioning compound, such as quaternized guar gum, which is a conventional thickening agent that is not a required aspect of the instant invention, and is preferably excluded from the instant invention.

WO 97/12020 relates to a liquid laundry detergent formulation comprising an anionic surfactant component and a quaternary ammonium compound component. The compositions of WO 97/12020 are not said to be clear or exhibit high viscosity and, as set forth in more detail below, formulations made according to the teachings of the Examples of WO 97/12020 are not clear.

### SUMMARY OF THE INVENTION

The present invention is directed to novel formulations that are particularly useful for personal care products which includes, for example, hair conditioners, shampoos, two-in-one shampoo/conditioners, skin conditioners, body washes, liquid bath soaps, facial cleansers, make-up removers, baby baths, and hand soaps. The formulations of the present invention are clear and may be made thick, both of which are highly desirable and attractive properties for personal care products. Thus, the formulations of the present invention overcome the problems in the art discussed above, and also exhibits the properties and advantages described herein.

One aspect of the present invention comprises a clear composition formulated from ingredients comprising:
(a) a primary anionic surfactant;
(b) a secondary surfactant; and
(c) a nitrogen-containing compound of structural formula (1): 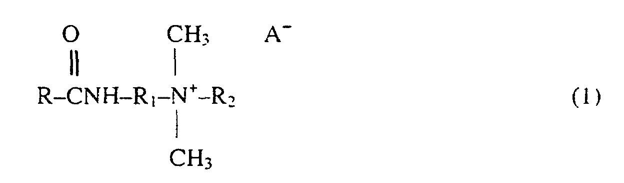
wherein
R is a saturated or unsaturated, linear, branched or cyclic alkyl or aryl group containing 12 to 22 carbon atoms that is unsubstituted;
R₁ is a saturated or unsaturated, linear, branched or cyclic alkylene group containing 1 to 6 carbon atoms and 0 to 3 hydroxyl groups;
R₂ is selected from the group consisting of hydrogen, methyl, and benzyl; and
A⁻ is a monovalent anion.

In a preferred embodiment of the invention, at least 50%, more preferably at least 65%, even more preferably at least 80%, and most preferably 90%, of the total amount the nitrogen-containing compounds comprises nitrogen-containing compounds having a common R group (that is, of the same chain length or, more preferably of the same group).

As used herein, the term "unsubstituted" means that the group does not contain any atoms other than carbon and hydrogen within the group.

As used herein, the term "primary anionic surfactant" is intended to include any anionic surfactant, such as ammonium lauryl sulfate, sodium lauryl sulfate, any α-olefin sulfonate, ammonium laureth sulfate (2 or 3 moles EO), sodium laureth sulfate (2 or 3 moles EO), sodium myristyl sulfate, sodium myristeth sulfate (1-4 moles EO), TEA-dodecylbenzene sulfonate, TEA lauryl sulfate, ammonium pareth sulfate, sodium pareth sulfate, sodium oleth sulfate, derivatives of any of the forgoing, and similar compounds known to those of skill in the art, and mixtures thereof.

As used herein, the term "secondary surfactant" is intended to include amphoteric surfactants. non-ionic surfactants, betaines, sulfosuccinates, mono- and diglycerides, glycinates, sugars and derivatives thereof, hydroxysultaines, mono- and diacetates, ethoxylated derivatives of any of the forgoing, and similar compounds known to those of skill in the art, and mixtures thereof. The purpose of the secondary surfactant is to enhance the charge interaction in such a way that no precipitation occurs and any surfactant that accomplishes or would be expected to accomplish this result may be used as the secondary surfactant in the invention. Although not limited to the following, the hydrophobe moieties of the secondary surfactant may be derived from any of the following whole oils or mixtures thereof: tallow, jojoba, palm, coconut, avocado, babassu, wheat germ, rapeseed, olive, orange, corn, linseed, neem, peanut, safflower, sesame seed, soybean, sunflower seed, and cocoa butter.

Another aspect of the present invention comprises a composition formulated from ingredients comprising:
(a) a primary anionic surfactant;
(b) a secondary surfactant; and
(c) a nitrogen-containing compound of the structural formula (2): 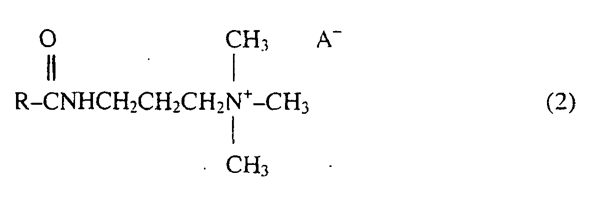
wherein
R is a saturated or unsaturated, linear, branched or cyclic alkyl or aryl group containing 12 to 22 carbon atoms that is unsubstituted; and
A⁻ is a monovalent anion.

The primary anionic surfactant is selected from the group consisting of ammonium lauryl sulfate, sodium lauryl sulfate, an α-olefin sulfonate, ammonium laureth sulfate (2 or 3 moles EO), sodium laureth sulfate (2 or 3 moles EO), sodium myristyl sulfate, sodium myristeth sulfate (1-4 moles EO), TEA-dodecylbenzene sulfonate, TEA lauryl sulfate, ammonium pareth sulfate, sodium pareth sulfate, sodium oleth sulfate, derivatives of any of the forgoing, and mixtures thereof. The primary anionic surfactant comprises between 5 wt.% to 50 wt.% (expressed as actives) of the total amount of components (a), (b), and (c), expressed as actives. In a preferred embodiment of the present invention, the primary anionic surfactant (as actives) comprises between 10 wt.% to 35 wt.% (expressed as actives) of the total amount of components (a), (b), and (c), expressed as actives. In a further preferred embodiment of the present invention, the primary anionic surfactant (as actives) comprises between 15 wt.% to 25 wt.% (expressed as actives) of the total amount of components (a), (b), and (c), expressed as actives.

The secondary surfactant is selected from the group consisting of cocamidopropyl betaine, lauramidopropyl betaine, ricinoleamidopropyl betaine, myristamidopropyl betaine. palmamidopropyl betaine, stearamidopropyl betaine, behenamidopropyl betaine. erucamidopropyl betaine, cocamidopropyl hydroxysultaine, myristamidopropyl hydroxysultaine, palmamidopropyl hydroxysultaine, stearamidopropyl hydroxysultaine, behenamidopropyl hydroxysultaine, erucamidopropyl hydroxysultaine, disodium lauroamphodiacetate, disodium cocamphodiacetate, disodium myristamphodiacetate, disodium palmamphodiacetate, disodium stearamphodiacetate, disodium behenamphodiacetate, disodium erucamphodiacetate, sodium lauryl amphoacetate, sodium cocamphoacetate, sodium cocoamphopropionate, sodium laurylamphopropionate, disodium lauroamphodipropionate, sodium lauryl sulfosuccinate, disodium laureth sulfosuccinate, cocobetaine, laurylbetaine, myristylbetaine, stearylbetaine, behenylbetaine, PEG 1-300 glyceryl cocoate, such as PEG 200 glyceryl cocoate, PEG 1-300 glyceryl tallowate, PEG 1-500 hydrogenated glyceryl palmitate, coco-glucoside, lauryl glucoside, decyl glucoside, and mixtures thereof. The secondary surfactant comprises between 5 wt.% to 50 wt.% (expressed as actives) of the total amount of components (a), (b), and (c), expressed as actives. In a preferred embodiment of the present invention, the secondary surfactant comprises between 5 wt.% to 30 wt.% (expressed as actives) of the total amount of components (a), (b), and (c), expressed as actives. In a further preferred embodiment of the present invention, the secondary surfactant comprises between 10 wt.% to 20 wt.% (expressed as actives) of the total amount of components (a), (b), and (c), expressed as actives.

The nitrogen-containing compound (which may be of formula (1) and/or (2)) comprises between 1.5 wt.% to 50 wt.% (expressed as actives) of the total amount of components (a), (b), and (c), expressed as actives. In a preferred embodiment of the present invention, the nitrogen-containing compound comprises between 2 wt.% to 20 wt.% (expressed as actives) of the total amount of components (a). (b), and (c), expressed as actives. In a further preferred embodiment of the present invention, the nitrogen-containing compound comprises between 2 wt.% to 10 wt.% (expressed as actives) of the total amount of components (a), (b), and (c), expressed as actives. Examples of the nitrogen-containing compounds according to the instant invention include palmitamidopropyltrimonium chloride, behenamidopropyl trimonium chloride, cetylamidopropyltrimonium chloride, palmitamidopropyltrimonium bromide, stearylamidopropyl methosulfate. tallowamidoproplytrimonium chloride, soyamidopropyltrimonium chloride, and canolamidopropyltrimonium methosulfate.

In a preferred embodiment of the invention, the composition further comprises a salt, the salt may be selected from the group consisting of: sodium chloride, potassium chloride, calcium chloride, magnesium chloride, ammonium chloride, sodium bromide, potassium bromide, calcium bromide, magnesium bromide, ammonium bromide, sodium iodide, potassium iodide, calcium iodide, magnesium iodide, ammonium iodide, sodium acetate, potassium acetate, or mixtures thereof. The salt component, if present, may be used to modify the viscosity of the resulting composition. In a further preferred embodiment of the present invention, the salt component comprises between 1 wt.% to 50 wt.%, more preferably between 2 wt.% to 20 wt.%, and most preferably between 2 wt.% to 10 wt.% of the total amount of components (a), (b), and (c), expressed as actives. In some embodiments of the invention, the composition does not contain a significant or any salts.

In certain embodiments of the present invention, the composition does not contain a significant amount or any conventional thickening agents. The term "conventional thickening agents" as used herein means any thickening agent or viscosity-enhancing agent known to those of skill in the art, excepting salts. Examples of such conventional thickening agents include quaternized guar gum, hydroxypropyl-substituted guar gum (such as that available from Rhône-Poulenc Corporation under the tradename JAGUAR@ HP200), polyethylene glycol (such as that available from Union Carbide Corporation under the tradename CARBOWAX® 20M), hydrophobic modified hydroxyethylcellulose (such as that available from the Aqualon Company under the tradename NATROSOL® Plus), and organophilic clays.

### BRIEF DESCRIPTION OF THE DRAWINGS

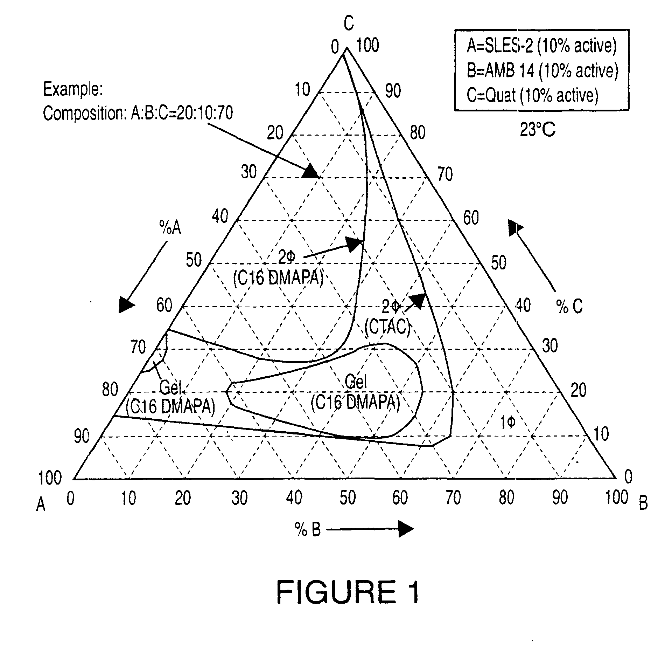

Figure 1 is a phase diagram illustrating the contrast between two ternary systems having components A, B, and C, wherein component A is sodium laureth sulfate (SLES-2), component B is cocamidopropyl betaine (AMB 14), and component C is either a dimethyl amidopropyl amine-based (DMAPA-based) quat or cetyl trimethyl ammonium chloride (CTAC).

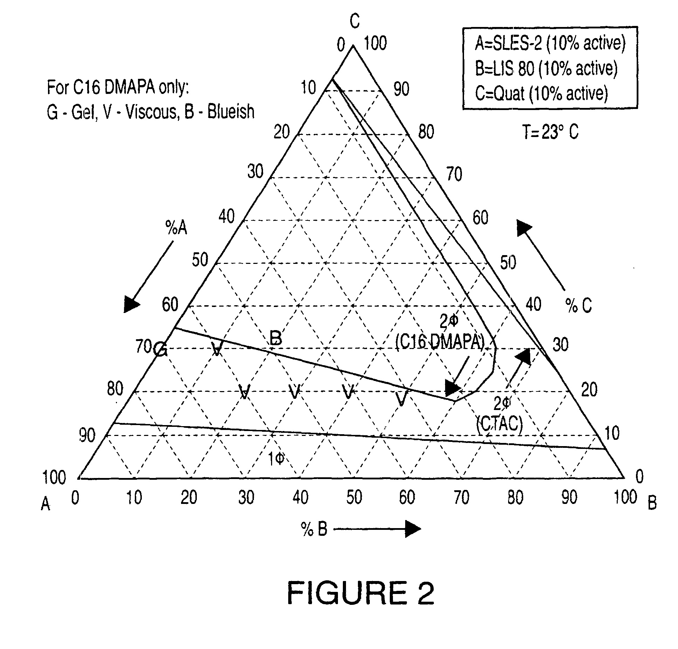

Figure 2 is a phase diagram illustrating the contrast between two ternary systems having components A, B, and C. wherein component A is sodium laureth sulfate (SLES-2), component B is PEG-200 hydrogenated glyceryl palmate/PEG-7 glyceryl cocoate (LI S 80), and component C is either a DMAPA-based quat or CTAC.

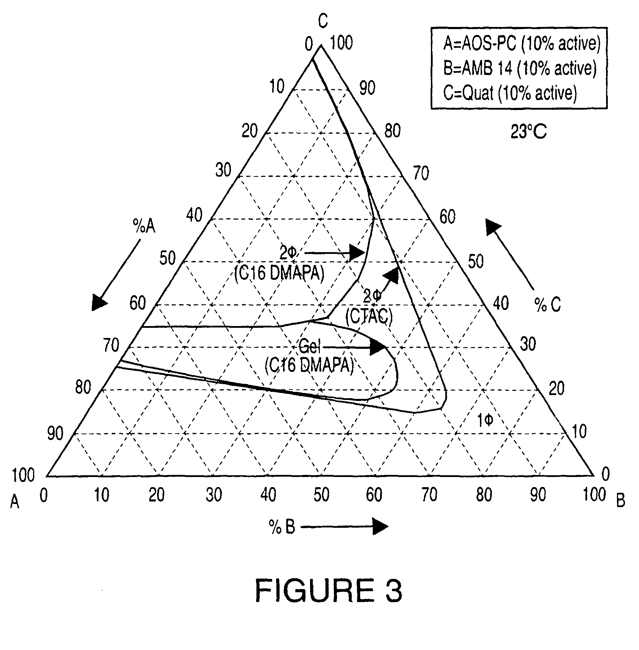

Figure 3 is a phase diagram illustrating the contrast between two ternary systems having components A, B, and C, wherein component A is C14-16 α-olefin sulfonate (AOS-PC), component B is cocamidopropyl betaine (AMB 14), and component C is either a DMAPA-based quat or CTAC.

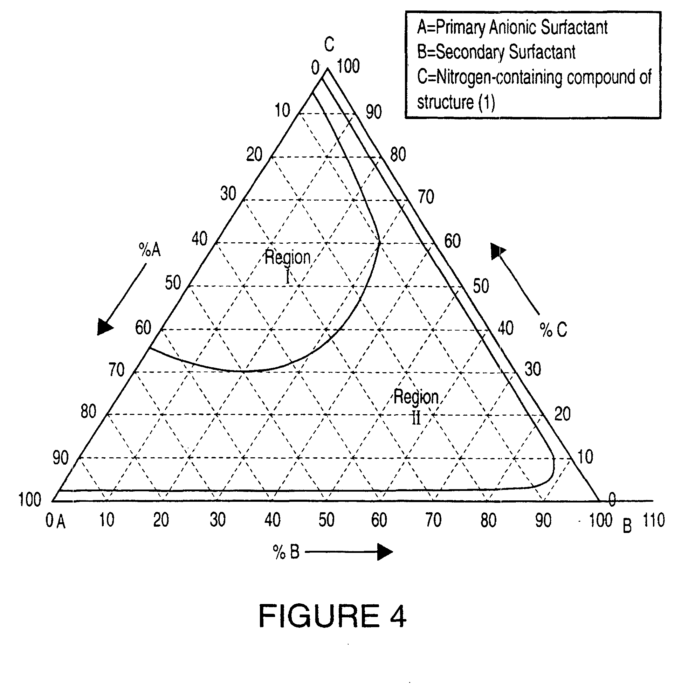

Figure 4 is a model phase diagram illustrating the scope of the compositions of the present invention within the one phase and gel Region II, showing the ranges of components A, B, and C, wherein component A is the primary anionic surfactant, component B is the secondary surfactant, and component C is the nitrogen-containing compound of the structural formula (1).

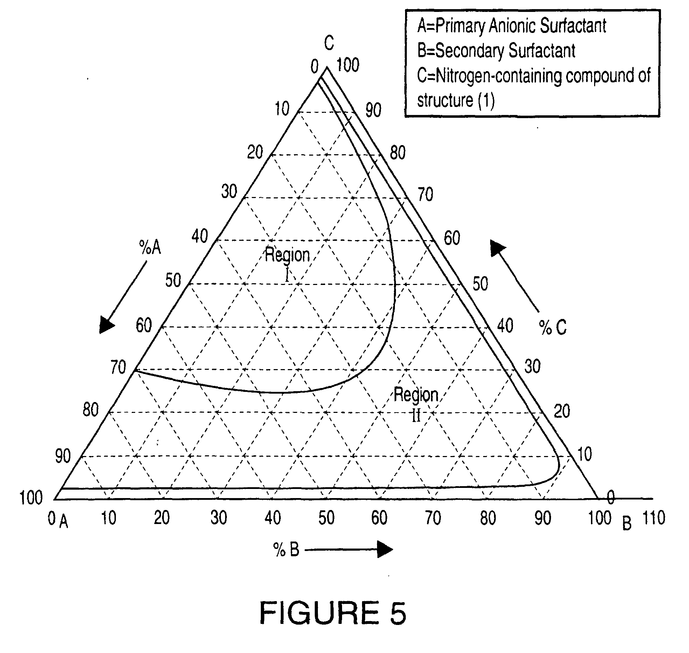

Figure 5 is a model phase diagram illustrating the preferred scope of the compositions of the present invention within the one phase and gel Region II, showing the ranges of components A, B, and C, wherein component A is the primary anionic surfactant, component B is the secondary surfactant, and component C is the nitrogen-containing compound of the structural formula (1).

### DETAILED DESCRIPTION OF THE INVENTION

The present invention is directed to novel formulations that arc particularly useful for personal care products which includes, for example, hair conditioners, shampoos, two-in-one shampoo/conditioners, skin conditioners, body washes, liquid bath soaps, facial cleansers, make-up removers, baby baths, and hand soaps. The formulations of the present invention are clear and also show unexpected viscosity building properties and may be made thick, both highly desirable and attractive properties for personal care products. The hair conditioner and two-in-one shampoo/conditioners formulations of the present invention exhibits good static decay reduction of the hair tress and exceptional conditioning feel.

Examples and experiments were conducted to illustrate the present invention. These examples are intended only to be illustrative of the present invention and are not intended to limit the present invention. The wt.% of ingredients shown in the Examples below is the amount of the ingredient weighed out as obtained and added to the formulation; in order to determine the amount of each active ingredient, that is, the wt.% of that component expressed as actives ("wt.% (as actives)"), use the percentage of the actives of that component and multiply by the wt.% given. For example, when sodium laureth sulfate (2 moles EO) (26% actives) is specified as 50.0 wt.% of the formulation, only (50.0 x 0.26) or 13 wt.% (as actives) of sodium laureth sulfate is present in the formulation.

### Examples

Formulations A-E below were prepared by measuring the individual ingredients into a vessel and mixing the resulting mixture at room temperature until it turned clear. The C16 DMAPA-based quat in Formulations A-E is palmitamidopropyltrimonium chloride. As used herein, the prefix attached to the DMAPA-based quat, for example, C16 or C12, designating the average number of carbon atoms in the R group of formula (1) and (2). Note that the "C16" designation is not meant to imply that all of the chain lengths are C 16, as commercial grade fatty acids derived from natural sources are a mixture of various chain lengths; "C16" is therefore meant to indicate the average chain length of the R group. As noted below, the pH of the resulting formulations may be adjusted by the addition of, for example, sodium hydroxide or citric acid. Perfumes, preservatives, dyes and other additives may also be added, and the amount of sodium chloride can be varied to adjust the viscosity. The term "actives" is well-known to those of skill in the art and denotes the amount of actual substance of interest in a given carrier, such as water or propylene glycol, and is usually expressed in percent. Thus, adding 1 gram of C 16 DMAPA-based quat solution (30% actives) contributes 0.3 grams of C16 DMAPA-based quat to the formulation.

| **FORMULATION A** | |
|---|---|
| **Ingredient** | **Wt.%** |
| Sodium laureth sulfate (2 mole EO) (26% actives) | 50.0 |
| C16 DMAPA-based quat (30% actives) | 6.6 |
| PEG-200 hydrogenated glyceryl palmate/PEG-7 glyceryl cocoate (70% actives) | 2.57 |
| Sodium chloride | 1.0 |
| Deionized water | qs to 100 |

| **FORMULATION B** | |
|---|---|
| **Ingredient** | **Wt.%** |
| Sodium laureth sulfate (2 mole EO) (26% actives) | 38.46 |
| C16 DMAPA-based quat (30% actives) | 16.67 |
| Cocamidopropyl betaine (35% actives) | 14.29 |
| Deionized water | qs to 100 |

| **FORMULATION C** | |
|---|---|
| **Ingredient** | **Wt.%** |
| Sodium laureth sulfate (2 mole EO) (26% actives) | 50.0 |
| C16 DMAPA-based quat (30% actives) | 6.6 |
| Cocamidopropyl betaine (35% actives) | 5.1 |
| Sodium chloride | 1.0 |
| Deionized water | qs to 100 |

| **FORMULATION D** | |
|---|---|
| **Ingredient** | **Wt.%** |
| Sodium laureth sulfate (2 mole EO) (26% actives) | 50.0 |
| C16 DMAPA-based quat (30% actives) | 6.6 |
| Cocamidopropyl hydroxysultaine (48% actives) | 3.75 |
| Sodium chloride | 1.0 |
| Deionized water | qs to 100 |

| **FORMULATION E** | |
|---|---|
| **Ingredient** | **Wt.%** |
| α-Olefin sulfonate (39% actives) | 33.3 |
| C16 DMAPA-based quat (30% actives) | 6.6 |
| Cocamidopropyl betaine (35% actives) | 5.1 |
| Sodium chloride | 1.0 |
| Deionized water | qs to 100 |

Formulations A-E demonstrate that various combinations of a C16 DMAPA-based quat, specifically, palmamidopropyltrimonium chloride, with sulfates or sulfonates in combination with betaines, hydroxysultaines, or ethoxylated mono- and diglycerides, produce clear systems whose viscosity may be readily adjusted.

Comparative Formulations X and Y below were made using the same procedure used in making Formulations A-E.

| **COMPARATIVE FORMULATION X** | |
|---|---|
| **Ingredient** | **Wt.%** |
| Sodium laureth sulfate (2 mole EO) (26% actives) | 50.0 |
| Cetyl trimethyl ammonium chloride (CTAC) (25% actives) | 8.0 |
| Cocamidopropyl betaine (35% actives) | 5.1 |
| Sodium chloride | 1.0 |
| Deionized water | qs to 100 |

| **COMPARATIVE FORMULATION Y** | |
|---|---|
| **Ingredient** | **Wt.%** |
| Sodium laureth sulfate (2 mole EO) (26% actives) | 50.0 |
| C16 DMAPA-based quat (30% actives) | 6.6 |
| Cocamidopropyl betaine (35% actives) | 5.1 |
| Sodium chloride | 1.0 |
| Deionized water | qs to 100 |

Comparative Formulation X did not become clear or thick with the addition of sodium chloride and demonstrates that a DMAPA-based quat is a critical component in a formulation possessing such properties.

Comparative Formulation Y as made was initially clear with 5% actives of the C16 DMAPA-based quat. When an additional 1% actives of the C 16 DMAPA-based quat (3.33 grams) was added, the solution became cloudy and then became clear. When a further 1% actives of the C16 DMAPA-based quat (3.33 grams) was added to the solution, however, the solution remained cloudy. This demonstrates that there is a unique relationship between the ratio of primary anionic surfactant to secondary surfactant to DMAPA-based quat in making a clear formulation.

Formulations F-K were made to demonstrate that these clear formulations could be made with other chain lengths of DMAPA-based quats. Three DMAPA-based quats were synthesized with various alkyl chain lengths: (1) a C12 DMAPA chloride quat in water (30.56% quat actives uncorrected; liquid state); (2) a C22 DMAPA chloride quat in a propylene glycol solvent system (47.36% quat actives uncorrected; nonviscous liquid state); and (3) a C18 DMAPA dimethyl sulfate quat (80% quat actives uncorrected; solid state). All three quats were formulated into clear systems in combination with an anionic surfactant and an amphoteric surfactant or ethoxylated mono- or diglyceride. Viscosity measurements were also taken with control Formulation I, containing no quat, to show the thickening properties of the DMAPA-based quats.

| **FORMULATION F** | |
|---|---|
| **Ingredient** | **Wt.%** |
| Sodium laureth sulfate (2 mole EO) (26% actives) | 48.1 |
| C18 DMAPA-based DMS quat (80% actives) | 2.5 |
| Cocamidopropylbetaine (35% actives) | 5.1 |
| Deionized water | 43.3 |
| Sodium chloride | 1.0 |

All ingredients were combined at room temperature, the blend was heated until the quat dissolved and the formulation was even. Formulation F is clear.

| **FORMULATION G** | |
|---|---|
| **Ingredient** | **Wt.%** |
| Sodium laureth sulfate (2 mole EO) (26% actives) | 48.1 |
| C22 DMAPA-based quat in propylene glycol (47.36% actives) | 4.2 |
| Cocamidopropylbetaine (35% actives) | 5.1 |
| Deionized water | 41.6 |
| Sodium chloride | 1.0 |

All ingredients were combined at room temperature and mixed until even. Formulation G was slightly hazy initially but then went clear.

| **FORMULATION H** | |
|---|---|
| **Ingredient** | **Wt.%** |
| Sodium laureth sulfate (2 mole EO) (26% actives) | 48.1 |
| C12 DMAPA-based quat (30.56% actives) | 6.5 |
| Cocamidopropylbetaine (35% actives) | 5.1 |
| Deionized water | 39.3 |
| Sodium chloride | 1.0 |

All ingredients were combined at room temperature and mixed until even. Formulation H was slightly hazy initially but then went clear.

| **FORMULATION I** | |
|---|---|
| **Ingredient** | **Wt.%** |
| Sodium laureth sulfate (2 mole EO) (26% actives) | 48.1 |
| Cocamidopropylbetaine (36% actives) | 5.1 |
| Deionized water | 45.8 |
| Sodium chloride | 1.0 |

Formulation I was made as a control formulation. All ingredients were combined at room temperature and mixed until even and the resulting formulation is clear. The viscosity of the above formulations were measured with a Brookfield DV2 RVT viscometer (available from Brookfield Engineering Laboratories, Inc.), Spindle #6, 10 rpm, 25°C and the results are shown in Table 1.

**TABLE I**

| **Formulation** | **Viscosity (cps)** |
|---|---|
| Formulation F (C18 DMAPA-based quat formulation) | 19,000 |
| Formulation G (C22 DMAPA-based quat formulation) | 22,100 |
| Formulation H (C 12 DMAPA-based quat formulation) | 38,300 |
| Formulation I (control formulation) | 300 |

The result presented in Table I is surprising, as the formulation based on DMAPA-quats having a shorter chain length (Formulation H) has a higher viscosity than the formulation based on DMAPA-quats having a longer chain length (e.g., Formulation G). It has been determined that the viscosity of a particular formulation according to the instant invention is enhanced by using the nitrogen-containing compounds of formula (1) where the R groups have the same chain length or consist of the same groups. Thus, if a particular formulation according to the invention contains nitrogen containing compounds, the viscosity will increase as the chain length distribution decreases.

Formulations containing only the primary anionic surfactant (in this case, SLES-2) and C12, C16, and C22 nitrogen-containing compounds of formula (1) according to the present invention were also prepared. From these formulations it was found that such compositions of the present invention (a) provide significant viscosity without the addition of salt or conventional thickening agent; (b) exhibit a rapid and significant increase in viscosity with the addition of salt; (c) maintain such high viscosity at elevated temperatures; (d) exhibit an increase in viscosity with increase temperature (from 20°C to 30°C). A similar formulation using a quat outside the scope of the claimed invention (such as that sold by Witco Surfactant GmbH under the tradename REWOQUAT® RTM50) do not display any of these characteristics. Moreover, the composition according to the present invention mitigated the skin-irritating effects of the primary anionic surfactant and nitrogen-containing compounds of formula (1) in the formulation, which was particularly marked when the mole ratio of the primary anionic surfactant to the nitrogen-containing compounds of formula (1) was between about 3:1 to about 1:3; preferably between about 2:1 to about 1:2; more preferably between about 5:3 to about 3:5; and most preferably between about 3:2 to about 2:3. This seems to indicate that a complex is formed between these two components and the effects are expected to be present in the formulations according to the instant invention that also contain a secondary surfactant.

Additional Formulations J and K were made to show that the various alkyl chain lengths of the DMAPA-based quats could form clear systems in mono/diglycerides and diacetates:

| **FORMULATION J** | |
|---|---|
| **Ingredient** | **Wt.%** |
| Sodium laureth sulfate (2 mole EO) (26% actives) | 22.2 |
| C22 DMAPA-based quat in propylene glycol (47.36% actives) | 6.3 |
| Disodium cocoamphodiacetate (50% actives) | 10.0 |
| Deionized water | 61.5 |

All ingredients were combined at room temperature and mixed until even. Formulation J was slightly hazy initially but then went clear and is a gel.

| **FORMULATION K** | |
|---|---|
| **Ingredient** | **Wt.%** |
| Sodium laureth sulfate (2 mole EO) (26% actives) | 22.2 |
| C12 DMAPA-based quat in water (30.56% actives) | 9.82 |
| PEG-200 hydrogenated glyceryl palmate/PEG-7 glyceryl cocoate (70% actives) | 10.0 |
| Deionized water | 60.84 |

All ingredients were combined at room temperature and mixed until even. Formulation K was slightly hazy initially but then went clear and is slightly viscous.

### Example Formulation According to WO 97/12020

In order to test the performance of the compositions of the instant invention over the formulations prepared in WO 97/12020, the formulation A of Example III in WO 97/12020 was attempted, substituting similar ingredients where the specified ingredients were not available or particularly set forth; however, the following ingredients specified for formulation A of Example III was not incorporated into the formulation because they could not be obtained or it was unclear what they were: diethylenetriamine pentamethylene phosphonic acid (specified as 1.0 wt.%); Endo A (5000 CEVU/g) (specified as 0.05 wt.%); terephthalate based polymer (specified as 0.5 wt.%); and DC 3225C (specified as 0.04 wt.%); these unincorporated ingredients account for less than 2% of the formulation and they would not be expected to affect the clarity or opaqueness of the final product.

| **COMPARATIVE FORMULATION WO 97/12020** | | | |
|---|---|---|---|
| **Blend** | **Ingredient used [ingredient specified in WO 97/12020 (if different from that used)]** | **Wt.%** | **Amount used (g)** |
| A | SLES-3 (Witcolate ES-3) [C12-15 alkyl ethoxylated sulfate] | 2.0 | 6.0 |
| A | SLS (Witcolate WAC LA) [C12-15 alkyl sulfate] | 17.0 | 51.0 |
| A | Citric Acid Anhydrous | 1.0 | 3.0 |
| A | Ethanol | 1.8 | 5.4 |
| A | Boric Acid | 2.4 | 7.2 |
| A | DI Water | 26.16 | 78.48 |
| B | Varisoft PATC | 2.0 | 6.0 |
| B | C12-14 N-methyl glucamide | 5.0 | 15.0 |
| B | Alfonic 1214 EO 4.7 [C12-14 fatty alcohol ethoxylate] | 6.0 | 18.0 |
| B | Industrene 7018 [C12-18 fatty acid] | 11.0 | 33.0 |
| B | Monoethanolamine (98%) | 8.0 | 24.0 |
| B | Sodium Hydroxide | 1.0 | 3.0 |
| B | Propanediol | 14.5 | 43.5 |
| B | 2-Butyl-1-octanol [2-Butyl Octanol] | 1.0 | 3.0 |
| B | UCT Polydimethylsiloxane: T-structure branch terminus PS-410KG [Branched Silicone] | 0.3 | 0.9 |
| C | Termanyl 300 LDX [Amylase 300 KNU/g] | 0.1 | 0.3 |
| C | Lipolase 100L [Lipase D96/L 100 KNU/g] | 0.15 | 0.45 |
| C | Savinase 16.0 L-type EX [Protease 35 g/L] | 0.5 | 1.5 |
| C | Carenzyme 4500G [Carenzyme 5000 CEVU/g] | 0.09 | 0.27 |

Blend A ingredients were combined in a vessel and heated over a steam bath to approximately 80°C. Blend B ingredients were combined in a separate vessel and also heated over a steam bath to approximately 80°C. Blend B was then removed from heat, attached to a mixer and stirred while Blend A was slowly added to Blend B to form Blend A+B. The first time this Blend A+B cooled down, the glucamide separated from the rest of the blend. The Blend A+B was reheated in a water bath to approximately 80°C and stirred with cooling. Blend C was added when the Blend A+B reached room temperature to form Blend A+B+C. The resulting Blend A+B+C (a formulation according to the teachings of WO 97/12020) is a creamy, opaque emulsion and is not clear. This is probably primarily due to the significant proportion of fatty alcohol ethoxylate and fatty acid, where the former acts as an emulsifying agent and the latter which, along with other ingredients found in the formulation, acts as an opacifying agent and thickener.

### Ternary Phase Diagrams

Six ternary phase diagrams were prepared at 23°C to illustrate the difference in performance between a certain C16 DMAPA-based quat (palmitamidopropyltrimonium chloride) and cetyl trimethyl arnmonium chloride or cetrimonium chloride (CTAC) (available from Witco Corporation under the tradename VARISOFT® 300). These six ternary phase diagrams are presented in Figures 1 to 3, where each Figure contains two phase diagrams. Each Figure, therefore, contains data for two separate ternary diagrams or systems: components A and B, which are the same for the two systems in a particular Figure, and component C, which is either the C16 DMAPA-based quat (in one system) or CTAC (in the other system). Thus, ternary systems 1 and 2 are illustrated in Figure 1, ternary systems 3 and 4 are illustrated in Figure 2, and ternary systems 5 and 6 are illustrated in Figure 3. The three components of each these systems is set forth in Table 2.

**TABLE 2**

| | **FIG. 1** | | **FIG. 2** | | **FIG. 3** | |
|---|---|---|---|---|---|---|
| **INCI Name (abbreviation)** | **1** | **2** | **3** | **4** | **5** | **6** |
| Sodium laureth sulfate (SLES-2) | X | X | X | X | | |
| Cocamidopropyl betaine (AMB 14) | X | X | | | X | X |
| Trimethylamidopropyl ammonium chloride (C16 DMAPA-based quat) | X | | X | | X | |
| Cetyl trimethyl ammonium chloride (CTAC) | | X | | X | | X |
| Sodium C14-16 α-olefin sulfonate (AOS-PC) | | | | | X | X |
| PEG-200 hydrogenated glyceryl palmate/PEG-7 glyceryl cocoate (LI S 80) | | | X | X | | |

As is typical of ternary phase diagrams, each comer of the phase diagram represents one species only (10% actives); for example, at comer C, only the quaternary compound or quat (either 10% C16 DMAPA-based quat or CTAC actives) is present. Moreover, there are boundary lines that indicate phase changes for each of the six ternary systems presented in Figures 1 to 3. The regions or composition ranges of a ternary system having a single phase are indicated by the symbol 1φ. The boundary line separating the region or composition range having one phase (1φ) and the region or composition range having two phases is indicated by an arrow having a legend with the symbol 2φ and indicating whether the boundary line applies to the DMAPA-based quat ternary system or the CTAC ternary system of that Figure. Additionally, any gel regions or compositions that exist for the ternary systems are labeled and it is indicated whether such gel regions apply to the DMAPA-based quat ternary system or the CTAC ternary system of that Figure.

In each of the experiments used to construct the ternary phase diagrams presented in Figures 1 to 3, a series of samples of 10 gram sample size were prepared in 20 mL vials. The anionic surfactants of the systems were always added last. Each sample was shaken by hand or, if necessary, by using a mechanical vibrator. Each sample was then put aside until the next day. The samples were then examined and the appearance of a phase boundary or gel phase or lack thereof was determined and such information was used to construct each ternary phase diagram. A sample was said to be two phase (2φ) if the sample showed a significantly bluish, turbid, or milky appearance.

It should be noted that the presence of gel or thickened phase regions in the C16 DMAPA-based quat ternary phase diagram is desirable for formulating products such as shampoos and body washes because it allows the formulators the freedom to choose desired viscosity.

### FIGURE 1

As shown in Table 2, Figure 1 contains ternary systems 1 and 2 which each have sodium laureth sulfate (SLES-2), available from Lonza, Inc. under the tradename CARSONOL® SLES-2, as component A, cocamidopropyl betaine (AMB 14), available from Witco Corporation under the tradename REWOTERIC® AMB 14. as component B, and either a C16 DMAPA-based quat (ternary system 1) or CTAC (ternary system 2) as component C. In Figure 1, the location of an example of a particular ternary composition (A:B:C=20:10:70), is indicated, including 20 wt.% of SLES-2, 10 wt.% of AMB 14, and 70 wt.% of either the C16 DMAPA-based quat (ternary system 1) or CTAC (ternary system 2). This particular composition indicated is in the two phase (2φ) region in both the C16 DMAPA-based quat and CTAC phase diagrams. Turning again to Figure 1, it can be seen that the corresponding ternary phase system of the C16 DMAPA-based quat is different from that of CTAC. As can be seen in Figure 1, the introduction of the DMAPA unit into the quat molecule changes the interaction between the quat and the anionic surfactant and unexpectedly leads to a richer phase behavior. Some important aspects of the difference between the behavior of the C16 DMAPA-based quat and CTAC discovered during these experiments and illustrated in Figure 1 may be summarized as follows:
a. The C16 DMAPA-based quat phase diagram has a much smaller two phase (2φ) region than that of the CTAC phase diagram.
b. The C16 DMAPA-based quat phase diagram has two gel phase regions (each very viscous), while the CTAC phase diagram has none.
c. The C16 DMAPA-based quat gel phases are clear.
d. The large two phase (2φ) region in the CTAC phase diagram contrasts with the two smaller gel phases and smaller two phase (2φ) region in the C16 DMAPA-based quat phase diagram.

### FIGURE 2

As shown in Table 2, Figure 2 contains ternary systems 3 and 4 which each have sodium laureth sulfate (SLES-2), available from Lonza, Inc. under the tradename CARSONOL® SLES-2, as component A, PEG-200 hydrogenated glyceryl palmate/PEG-7 glyceryl cocoate (LI S 80), available from Witco Corporation under the tradename VARONIC® LI S 80, as component B, and either the C16 DMAPA-based quat (ternary system 3) or CTAC (ternary system 4) as component C. As with Figure 1. it can be seen that the corresponding ternary phase system of the C16 DMAPA-based quat is different from that of CTAC. As can be seen in Figure 2, the introduction of a DMAPA unit into the quat molecule changes the interaction between the quat and the anionic surfactant and unexpectedly leads to a richer phase behavior. Some important aspects of the difference between the behavior of the C16 DMAPA-based quat and CTAC discovered during these experiments and illustrated in Figure 2 may be summarized as follows:
a. The C16 DMAPA-based quat phase diagram has a much smaller two phase (2φ) region than that of the CTAC phase diagram.
b. The C16 DMAPA-based quat phase diagram has one large viscous one phase (1φ) region.

The presence of the viscous region in the C16 DMAPA-based quat ternary phase diagram is desirable for formulating products such as shampoos and body washes because it allows the formulators the freedom to choose desired viscosity. The results showed that the thickening property for clear formulations (ternary mixtures) provided by the C16 DMAPA-based quat was unexpected and unique: this property was not found in the traditional quats.

### FIGURE 3

As shown in Table 2, Figure 3 contains ternary systems 5 and 6 which each have sodium C14-16 α-olefin sulfonate (AOS-PC), available from Rhone-Poulenc under the tradename RHODACAL® A-246-L, as component A, cocamidopropyl betaine (AMB 14), available from Witco Corporation under the tradename REWOTERIC® AMB 14, as component B, and either the C16 DMAPA-based quat (ternary system 5) or CTAC (ternary system 6) as component C. As with Figures 1 and 2, it can be seen that the corresponding ternary phase system of the C 16 DMAPA-based is different from that of CTAC. As can be seen in Figure 3, the introduction of the DMAPA unit into the quat molecule changes the interaction between the quat and the anionic surfactant and unexpectedly leads to a richer phase behavior. Some important aspects of the difference between the behavior of the C16 DMAPA-based quat and CTAC discovered during these experiments and illustrated in Figure 3 may be summarized as follows:
a. The C16 DMAPA-based quat phase diagram has a much smaller two phase (2φ) region than that of the CTAC phase diagram.
b. The C16 DMAPA-based quat phase diagram has one large gel phase region (very viscous) while the CTAC phase diagram has none.
c. The C16 DMAPA-based quat gel phase is clear.
d. The large two phase (2φ) region in the CTAC phase diagram contrasts with the one gel phase and smaller two phase (2φ) region in the C16 DMAPA-based quat phase diagram.
The presence of the gel phase region in the C16 DMAPA-based quat ternary phase diagram is desirable for formulating products such as shampoos and body washes because it allows the formulators the freedom to choose desired viscosity.

As noted above. oppositely charged species can form complexes (or ion pairs) in dielectric media. Depending on the extent of this charge interaction, it may lead to the formation of a precipitate in strongly interacting systems. It is well-known in the art that quaternized ammonium surfactants strongly interact with anionic surfactants, leading to precipitation or dispersible complexes. In either case, clear systems cannot be formulated with these surfactants. It was unexpected to discover that a quaternized surfactant, for example, the C16 DMAPA-based quat, can thicken the anionic/amphoteric systems while remaining clear, a property not seen in the traditional quats. The unexpected properties of the C16 DMAPA-based quat can be seen in the phase diagrams shown in Figures 1 to 3.

Two additional ternary phase diagrams are set forth in Figures 4 and 5 to illustrate the range of preferred compositions according to the instant invention. In a preferred embodiment of the invention, the composition of the instant invention is defined as the one phase and gel regions (Region II) shown in Figure 4, wherein the primary anionic surfactant (Component A), the secondary surfactant (Component B), and the nitrogen-containing compound (Component C) are present in respective ratios that define and are within the one-phase and gel regions (Region II). In another preferred embodiment of the invention, the composition of the instant invention is defined as the one phase and gel regions (Region II) shown in Figure 5, wherein the primary anionic surfactant (Component A), the secondary surfactant (Component B), and the nitrogen-containing compound (Component C) are present in respective ratios that define and are within the one-phase and gel regions (Region II). In each of Figures 4 and 5, Components A, B, and C are expressed as wt.% of actives.

The DMAPA-based quat compounds can be used alone or in mixtures in the formulations proposed, can be used in combination with other compounds or additives, or used as a formulation with other compounds or additives, depending on the intended use and the advantages and disadvantages attendant with each alternative application method. The compositions as described herein exhibit a number of desirable properties making them particularly suitable for formulation into commercial products such as conditioners and other personal care products as mentioned above. Most notably, the compositions are clear, i.e., transparent or translucent. This property can be realized at a variety of concentrations of active ingredient, with or even without special solvents or coupling agents. Other properties are realized as well. For instance, many of the compositions form a stable gel and exhibit advantageous stability, solubility, and freedom from objectionable color and odor.

It should be noted that the compositions of the present invention may be formulated without a solvent system, for example, as a solid or flake composition, or may be formulated to include water, propylene glycol, ethanol, isopropanol, diethylene glycol, or similar solvent of mixture thereof, as a concentrate or more dilute form, depending on the application. Selection of a suitable solvent for a particular application is well-known to those of skill in the art.

It is understood that the compositions of the present invention can also contain appropriate aethestic additives for a given application, as would be known to those of skill in the art including, without limitation, perfumes, preservatives, silicones, and dyes. Moreover, additives may be used to adjust the chemical or physical properties of the formulation to a desired level. Thus, an acid, for example, citric acid, may be used to adjust the pH of the composition to an appropriate level and sodium chloride or other salt may be used to adjust the viscosity of the composition to a desired level. Usually these additives are present in small amounts, generally up to 2 wt.% each. to provide the desired properties. Other compounds or additives familiar to those of skill in the art and appropriate to a particular use may also be used with or formulated with the composition of the present invention.

It is also understood that many of the examples and claims presented include components that are salts, that is, they include an anion and a cation. It is understood by those of skill in the art that the identity anion or cation of a given compound may not be crucial in the activity of the compound for a given purpose (that is, it may constitute a spectator ion) and an appropriate substitute may be made therefor. Thus, with regard to the DMAPA-based quats, the counteranion may be, for example, chloride, bromide, methyl sulfate, ethyl sulfate, or salicylate. Similarly, the sodium ion present in many of the primary anionic surfactants claimed and presented in the examples above may be replaced by other cations, such as potassium ion or ammonium ion. without appreciably affecting the performance of the primary anionic surfactant. Furthermore, with regard to the salt component that may be added to the composition or subsequently added to the composition to obtain a desired viscosity, the term "salt" is intended to cover all of the compounds known to those of skill in the art to be equivalent to those mentioned herein. Thus, it is understood that such ions may be substituted by any other ion which is not significantly deleterious to the desired chemical or physical properties of the overall compound in its intended use herein. It is therefore understood that such ion substitution is well-known in the art and all such possibilities and equivalents are intended to be embraced within the appended claims.

As noted above, the examples provided are intended to further describe the aspects of the present invention. The examples are illustrative only and are not to be construed as limiting the scope of that which is regarded as the invention. Therefore, the scope of the present invention is only to be limited by the following claims and the equivalents thereto.

## Claims

1. A composition formulated from ingredients comprising:
(a) 5 to 50 wt.% of a primary anionic surfactant, based on the composition, selected from the group consisting of ammonium lauryl sulfate, sodium lauryl sulfate, an α- olefin sulfonate, ammonium laureth sulfate (2 or 3 moles EO), sodium laureth sulfate (2 or 3 moles EO), sodium myristyl sulfate, sodium myristeth sulfate (1 - 4 moles EO), TEA-dodecylbenzene sulfonate, TEA lauryl sulfate, ammonium pareth sulfate, sodium pareth sulfate, sodium oleth sulfate, and mixtures thereof;
(b) 5 to 50 wt.% (expressed as actives) of a secondary surfactant, based on the total amount of compounds (a), (b) and (c), selected from the group consisting of: cocamidopropyl betaine, lauramidopropyl betaine, ricinoleamidopropyl betaine, myristamidopropyl betaine, palmamidopropyl betaine, stearamidopropyl betaine, behenamidopropyl betaine, erucamidopropyl betaine, cocamidopropyl hydroxysultaine, myristamidopropyl hydroxysultaine, palmamidopropyl hydroxysultaine, stearamidopropyl hydroxysultaine, behenamidopropyl hydroxysultaine, erucamidopropyl hydroxysultaine, disodium lauroamphodiacetate, disodium cocamphodiacetate, disodium myristamphodiacetate, disodium palmamphodiacetate, disodium stearamphodiacetate, disodium behenamphodiacetate, disodium erucamphodiacetate, sodium lauryl amphoacetate, sodium cocamphoacetate, sodium cocoamphopropionate, sodium laurylamphopropionate, disodium lauroamphodipropionate, sodium lauryl sulfosuccinate, disodium laureth sulfosuccinate, cocobetaine, laurylbetaine, myristylbetaine, stearylbetaine, behenylbetaine, PEG 1-300 glyceryl cocoate, PEG 1-300 glyceryl tallowate, PEG 1-500 hydrogenated glyceryl palmitate, coco-glucoside, lauryl glucoside decyl glucoside, and mixtures thereof; and
(c) 1.5 to 50 wt.% (expressed as actives) of a nitrogen-containing compound, based on the total amount of compounds (a), (b) and (c), said compound being of the structural formula: 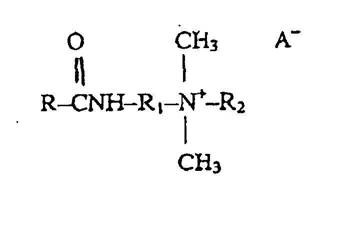 wherein
R is a saturated or unsaturated, linear, branched or cyclic alkyl or aryl group containing 12 to 22 carbon atoms that is unsubstituted;
R₁ is a saturated or unsaturated, linear, branched or cyclic alkylene group containing 1 to 6 carbon atoms and 0 to 3 hydroxyl groups;
R₂ is selected from the group consisting of hydrogen, methyl and benzyl; and
A is a monovalent anion.

2. The composition according to claim 1, wherein the secondary surfactant comprises between 5 wt.% to 30 wt.% (expressed as actives) of the total amount of components (a), (b) and (c), expressed as actives.

3. The composition according to claim 1, wherein the nitrogen-containing compound comprises between 2 wt.% to 20 wt.% (expressed as actives) of the total amount of components (a), (b) and (c), expressed as actives.

4. The composition according to claim 1, wherein the nitrogen-containing compound comprises between 2 wt.% to 10 wt.% (expressed as actives) of the total amount of components (a), (b) and (c), expressed as actives.

5. The composition according to claim 1, wherein R₁ is -CH₂CH₂CH₂-.

6. The composition according to claim 1, wherein R is a saturated, linear alkyl group containing 12 to 22 carbon atoms that is unsubstituted.

7. The composition according to claim 1, wherein R₂ is methyl.

8. The composition according to claim 1, further comprising:
(d) a salt.

9. The composition according to claim 8, wherein the salt is selected from the group consisting of: sodium chloride, potassium chloride, calcium chloride, magnesium chloride, ammonium chloride, sodium bromide, potassium bromide, calcium bromide, magnesium bromide, ammonium bromide, sodium iodide, potassium iodide, calcium iodide, magnesium iodide, ammonium iodide, sodium acetate, potassium acetate or mixtures thereof.

10. The composition according to claim 9, wherein R₁ is -CH₂CH₂CH₂-.

11. The composition according to claim 9, wherein R is a saturated, linear alkyl group containing 12 to 22 carbon atoms.

12. The composition according to claim 7, further comprising:
(d) a salt.

13. The composition according to claim 12, wherein the salt is selected from the group consisting of: sodium chloride, potassium chloride, calcium chloride, magnesium chloride, ammonium chloride, sodium bromide, potassium bromide, calcium bromide, magnesium bromide, ammonium bromide, sodium iodide, potassium iodide, calcium iodide, magnesium iodide, ammonium iodide, sodium acetate, potassium acetate or mixtures thereof.

14. The composition according to claim 1, wherein the primary anionic surfactant, the secondary surfactant, and the nitrogen-containing compound are present in respective ratios that define and are within the Region II of Figure 4, the primary anionic surfactant being component A of Figure 4, the secondary surfactant being component B of Figure 4, and the nitrogen-containing compound being component C of Figure 4.

15. The composition according to claim 1, wherein the primary anionic surfactant, the secondary surfactant, and the nitrogen-containing compound are present in respective ratios that define and are within the Region II of Figure 5, the primary anionic surfactant being component A of Figure 5, the secondary surfactant being component B of Figure 5, and the nitrogen-containing compound being component C of Figure 5.

## Patentansprüche

1. Zusammensetzung, formuliert aus Bestandteilen, umfassend:
(a) 5 bis 50 Gew.-% eines primären anionischen Tensids, bezogen auf die Zusammensetzung, ausgewählt aus der Gruppe bestehend aus Ammoniumlaurylsulfat, Natriumlaurylsulfat, einem α-Olefinsulfonat, Ammoniumlaurethsulfat (2 oder 3 mol EO), Natriumlaurethsulfat (2 oder 3 mol EO), Natriummyristylsulfat, Natriummyristethsulfat (1 bis 4 mol EO), TEA-Dodecylbenzolsulfonat, TEA-Laurylsulfat, Ammoniumparethsulfat, Natriumparethsulfat, Natriumolethsulfat und Mischungen davon,
(b) 5 bis 50 Gew.-% (ausgedrückt als aktive Bestandteile) eines zweiten Tensids, bezogen auf die Gesamtmenge der Verbindungen (a), (b) und (c), ausgewählt aus der Gruppe bestehend aus: Cocamidopropylbetain, Lauramidopropylbetain, Ricinolamidopropylbetain, Myristamidopropylbetain, Palmamidopropylbetain, Stearamidopropylbetain, Behenamidopropylbetain, Erucamidopropylbetain, Cocamidopropylhydroxysultain, Myristamidopropylhydroxysultain, Palmamidopropylhydroxysultain, Stearamidopropylhydroxysultain, Behenamidopropylhydroxysultain, Erucamidopropylhydroxysultain, Dinatriumlauroamphodiacetat, Dinatriumcocamphodiacetat, Dinatriummyristamphodiacetat, Dinatriumpalmamphodiacetat, Dinatriumstearamphodiacetat, Dinatriumbehenamphodiacetat, Dinatriumerucamphodiacetat, Natriumlaurylamphoacetat, Natriumcocamphoacetat, Natriumcocoamphopropionat, Natriumlaurylamphopropionat, Dinatriumlauroamphodipropionat, Natriumlaurylsulfosuccinat, Dinatriumlaurethsulfosuccinat, Cocobetain, Laurylbetain, Myristylbetain, Stearylbetain, Behenylbetain, PEG 1-300-Glycerylcocoat, PEG 1-300-Glyceryltalloat, hydriertes PEG 1-500-Glycerylpalmitat, Cocoglucosid, Laurylglucosid, Decylglucosid und Mischungen davon, und
(c) 1,5 bis 50 Gew.-% (ausgedrückt als aktive Bestandteile) einer Stickstoff enthaltenden Verbindung, bezogen auf die Gesamtmenge der Verbindungen (a), (b) und (c), wobei die Verbindung die Strukturförmel hat: 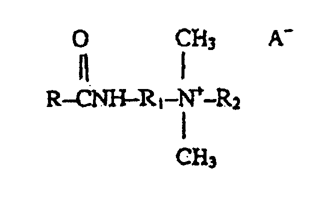
worin
R eine gesättigte oder ungesättigte, lineare, verzweigte oder cyclische Alkyl- oder Arylgruppe ist, die 12 bis 22 Kohlenstoffatome enthält, die unsubstituiert ist,
R₁ eine gesättigte oder ungesättigte, lineare, verzweigte oder cyclische Alkylengruppe ist, die 1 bis 6 Kohlenstoffatome und 0 bis 3 Hydroxylgruppen enthält,
R₂ ausgewählt ist aus der Gruppe bestehend aus Wasserstoff, Methyl und Benzyl, und
A⁻ ein einwertiges Anion ist.

2. Zusammensetzung gemäß Anspruch 1, worin das sekundäre Tensid zwischen 5 Gew.-% bis 30 Gew.-% (ausgedrückt als aktive Bestandteile) der Gesamtmenge der Komponenten (a), (b) und (c), ausgedrückt als aktive Bestandteile, umfasst.

3. Zusammensetzung gemäß Anspruch 1, worin die Stickstoff enthaltende Verbindung zwischen 2 Gew.-% bis 20 Gew.-% (ausgedrückt als aktive Bestandteile) der Gesamtmenge der Komponenten (a), (b) und (c), ausgedrückt als aktive Bestandteile, umfasst.

4. Zusammensetzung gemäß Anspruch 1, worin die Stickstoff enthaltende Verbindung zwischen 2 Gew.-% bis 10 Gew.-% (ausgedrückt als aktive Bestandteile) der Gesamtmenge der Komponenten (a), (b) und (c), ausgedrückt als aktive Bestandteile, umfasst.

5. Zusammensetzung gemäß Anspruch 1, worin R₁ -CH₂CH₂CH₂- ist.

6. Zusammensetzung gemäß Anspruch 1, worin R eine gesättigte, lineare Alkylgruppe ist, die 12 bis 22 Kohlenstoffatome enthält, die unsubstituiert ist.

7. Zusammensetzung gemäß Anspruch 1, worin R₂ Methyl ist.

8. Zusammensetzung gemäß Anspruch 1, weiter umfassend:
(d) ein Salz.

9. Zusammensetzung gemäß Anspruch 8, worin das Salz ausgewählt ist aus der Gruppe bestehend aus Natriumchlorid, Kaliumchlorid, Calciumchlorid, Magnesiumchlorid, Ammoniumchlorid, Natriumbromid, Kaliumbromid, Calciumbromid, Magnesiumbromid, Ammoniumbromid, Natriumiodid, Kaliumiodid, Calciumiodid, Magnesiumiodid, Ammoniumiodid, Natriumacetat, Kaliumacetat oder Mischungen davon.

10. Zusammensetzung gemäß Anspruch 9, worin R₁ -CH₂CH₂CH₂- ist.

11. Zusammensetzung gemäß Anspruch 9, worin R eine gesättigte, lineare Alkylgruppe ist, die 12 bis 22 Kohlenstoffatome enthält.

12. Zusammensetzung gemäß Anspruch 7, weiter umfassend:
(d) ein Salz.

13. Zusammensetzung gemäß Anspruch 12, worin das Salz ausgewählt ist aus der Gruppe bestehend aus Natriumchlorid, Kaliumchlorid, Calciumchlorid, Magnesiumchlorid, Ammoniumchlorid, Natriumbromid, Kaliumbromid, Calciumbromid, Magnesiumbromid, Ammoniumbromid, Natriumiodid, Kaliumiodid, Calciumiodid, Magnesiumiodid, Ammoniumiodid, Natriumacetat, Kaliumacetat oder Mischungen davon.

14. Zusammensetzung gemäß Anspruch 1, worin das primäre anionische Tensid, das sekundäre Tensid und die Stickstoff enthaltende Verbindung in Verhältnissen vorhanden sind, welche die Region II von Fig. 4 definieren und darin befindlich sind, wobei das primäre anionische Tensid die Komponente A von Fig. 4 ist, das sekundäre Tensid die Komponente B von Fig. 4 ist, und die Stickstoff enthaltende Verbindung die Komponente C von Fig. 4 ist.

15. Zusammensetzung gemäß Anspruch 1, worin das primäre anionische Tensid, das sekundäre Tensid und die Stickstoff enthaltende Verbindung in Verhältnissen vorhanden sind, welche die Region II von Fig. 5 definieren und darin befindlich sind, wobei das primäre anionische Tensid die Komponente A von Fig. 5 ist, das sekundäre Tensid die Komponente B von Fig. 5 ist, und die Stickstoff enthaltende Verbindung die Komponente C von Fig. 5 ist.

## Revendications

1. Composition formulée à partir d'ingrédients comprenant :
(a) 5 à 50% en poids, rapporté à la composition, d'un tensioactif anionique primaire, choisi dans le groupe formé par le laurylsulfate d'ammonium, le laurylsulfate de sodium, un sulfonate d'α-oléfine, le laureth-sulfate d'ammonium (2 ou 3 moles EO), le laureth-sulfate de sodium (2 ou 3 moles EO), le myristylsulfate de sodium, le myristeth-sulfate de sodium (1 à 4 moles EO), le TEA-dodécylbenzène-sulfonate, le TEA-laurylsulfate, le pareth-sulfate d'ammonium, le pareth-sulfate de sodium, l'oleth-sulfate de sodium et leurs mélanges ;
(b) 5 à 50% en poids (exprimés comme actifs) sur la base de la quantité totale des composés (a), (b) et (c), d'un tensioactif secondaire, choisi dans le groupe formé par : la bétaïne de cocamidopropyle, la bétaïne de lauramidopropyle, la bétaïne de ricinoléamidopropyle, la bétaïne de myristamidopropyle, la bétaïne de palmamidopropyle, la bétaïne de stéaramidopropyle, la bétaïne de béhénamidopropyle, la bétaïne d'érucamidopropyle, l'hydroxysultaïne de cocamidopropyle, l'hydroxysultaïne de myristamidopropyle, l'hydroxysultaïne de palmamidopropyle, l'hydroxysultaïne de stéaramidopropyle, l'hydroxysultaïne de béhénamidopropyle, l'hydroxysultaïne d'érucamidopropyle, le lauroamphodiacétate disodique, le cocamphodiacétate disodique, le myristamphodiacétate disodique, le palmamphodiacétate disodique, le stéaramphodiacétate disodique, le béhénamphodiacétate disodique, l'érucamphodiacétate disodique, le laurylamphoacétate de sodium, le cocamphoacétate de sodium, le cocoamphopropionate de sodium, le laurylamphopropionate de sodium, le lauroamphodipropionate disodique, le laurylsulfosuccinate de sodium, le laureth-sulfosuccinate disodique, la cocobétaïne, la laurylbétaïne, la myristylbétaïne, la stéarylbétaïne, la béhénylbétaïne, le cocoate de glycéryle PEG 1-300, le tallowate de glycéryle PEG 1-300, le PEG 1-500 palmitate de glycéryle hydrogéné, le coco-glucoside, le lauryl-glucoside, le décyl-glucoside et leurs mélanges ; et
(c) 1,5 à 50% en poids (exprimés comme actifs) sur la base d'une quantité totale des composés (a), (b) et (c), d'un composé contenant de l'azote, ledit composé ayant la structure de formule :
R-C(=O)NH-R₁-N⁺(CH₃)₂-R₂ A⁻
dans laquelle
R représente un groupe alkyle linéaire, ramifié ou cyclique, saturé ou insaturé ou un groupe aryle contenant 12 à 22 atomes de carbone, qui n'est pas substitué ;
Ri représente un groupe alkylène linéaire, ramifié ou cyclique, saturé ou insaturé, contenant 1 à 6 atomes de carbone et 0 à 3 groupes hydroxyle ;
R₂ est choisi dans le groupe comprenant un atome d'hydrogène, un groupe méthyle et un groupe benzyle, et A représente un anion monovalent.

2. Composition selon la revendication 1, dans laquelle le tensioactif secondaire représente entre 5% et 30% en poids (exprimés comme actifs) de la quantité totale des composants (a), (b) et (c), exprimés comme actifs.

3. Composition selon la revendication 1, dans laquelle le composé contenant de l'azote représente entre 2% et 20% en poids (exprimés comme actifs) de la quantité totale des composants (a), (b) et (c), exprimés comme actifs.

4. Composition selon la revendication 1, dans laquelle le composé contenant de l'azote représente entre 2% et 10% en poids (exprimés comme actifs) de la quantité totale des composants (a), (b) et (c), exprimés comme actifs.

5. Composition selon la revendication 1, dans laquelle Ri représente ―CH₂CH₂CH₂-.

6. Composition selon la revendication 1, dans laquelle R représente un groupe alkyle linéaire saturé contenant 12 à 22 atomes de carbone, qui est non substitué.

7. Composition selon la revendication 1, dans laquelle R₂ représente un groupe méthyle.

8. Composition selon la revendication 1, comprenant en outre :
(d) un sel.

9. Composition selon la revendication 8, dans laquelle le sel est choisi dans le groupe formé par : le chlorure de sodium, le chlorure de potassium, le chlorure de calcium, le chlorure de magnésium, le chlorure d'ammonium, le bromure de sodium, le bromure de potassium, le bromure de calcium, le bromure de magnésium, le bromure d'ammonium, l'iodure de sodium, l'iodure de potassium, l'iodure de calcium, l'iodure de magnésium, l'iodure d'ammonium, l'acétate de sodium, l'acétate de potassium ou leurs mélanges.

10. Composition selon la revendication 9, dans laquelle R₁ représente -CH₂CH₂CH₂-.

11. Composition selon la revendication 9, dans laquelle R représente un groupe alkyle linéaire saturé contenant 12 à 22 atomes de carbone.

12. Composition selon la revendication 7, comprenant en outre :
(d) un sel.

13. Composition selon la revendication 12, dans laquelle le sel est choisi dans le groupe formé par : le chlorure de sodium, le chlorure de potassium, le chlorure de calcium, le chlorure de magnésium, le chlorure d'ammonium, le bromure de sodium, le bromure de potassium, le bromure de calcium, le bromure de magnésium, le bromure d'ammonium, l'iodure de sodium, l'iodure de potassium, l'iodure de calcium, l'iodure de magnésium, l'iodure d'ammonium, l'acétate de sodium, l'acétate de potassium ou leurs mélanges.

14. Composition selon la revendication 1, dans laquelle le tensiioactif anionique primaire, le tensioactif secondaire et le composé contenant de l'azote sont présents dans des rapports respectifs qui définissent et se situent dans la Région II de la figure 4, le tensioactif anionique primaire étant le composant A de la figure 4, le tensioactif secondaire étant le composant B de la figure 4 et le composé contenant de l'azote étant le composant C de la figure 4.

15. Composition selon la revendication 1, dans laquelle le tensioactif anionique primaire, le tensioactif secondaire et le composé contenant de l'azote sont présents dans des rapports respectifs qui définissent et se situent dans la Région II de la figure 5, le tensioactif anionique primaire étant le composant A de la figure 5, le tensioactif secondaire étant le composant B de la figure 5 et le composé contenant de l'azote étant le composant C de la figure 5.
